# EUROPÄISCHE PATENTANMELDUNG

(11) **EP 3 072 443 A1**
(43) Veröffentlichungstag der Anmeldung: **28.09.2016**
(21) Anmeldenummer: 16152164.6
(22) Anmeldetag: 21.01.2016
(51) Int. Cl.: A61B 5/022, A61B 5/024, A61B 5/00

(54) **BDM-SYSTEM FÜR DIE LANGZEITMESSUNG EINES BLUTDRUCKS**

(30) Priorität: 24.03.2015 DE 102015104432
(71) Anmelder: Beurer GmbH, 89077 Ulm (DE)
(72) Erfinder: Bühler, Marco, 89075 Ulm (DE); Stegmaier, Sylvia, 89077 Ulm (DE); Meternek, Werner, 89233 Neu-Ulm (DE); Lindner, Bernd, 73037 Göppingen (DE); Leist, Angela, 72353 Heroldstadt (DE); Strobel, Thomas, 86480 Waltenhausen (DE); Kebbe, Sebastian, 68642 Bürstadt (DE); Ott, Julia, 89073 Ulm (DE)
(74) Vertreter: Meitinger, Thomas Heinz

(57) **Zusammenfassung**

Ein System zur Messung des Blutdrucks eines Anwenders umfasst eine an einem Oberarm eines Anwenders befestigbare Manschettenvorrichtung (1) mit einem Sensor zur Messung eines Blutdrucks und einer Datenübeitragungsvorrichtung zur Übertragung der gemessenen Blutdruckdaten und eine an einem Handgelenk des Anwenders befestigbare Anzeigevorrichtung mit einer Datenempfangsvorrichtung zum Empfang der von der Datenübertragungsvorrichtung übertragenen Daten.

## Beschreibung

### GEBIET DER ERFINDUNG

Die vorliegende Erfindung betrifft ein System zur Messung des Blutdrucks eines Anwenders.

### HINTERGRUND DER ERFINDUNG

Im Stand der Technik sind Blutdruckmessgeräte (BDM) bekannt, die eine eigenständige Messung ermöglichen. Außerdem gibt es auch Messgeräte, die speziell für eine 24-stündige Messung vorgesehen sind.

### ZUSAMMENFASSUNG DER ERFINDUNG

Der Blutdruck eines Anwenders kann im Laufe eines Tages stark schwanken. Sinnvoll sind daher regelmäßige Messungen, die über den ganzen Tag verteilt sind. Hierdurch können repräsentative Messergebnisse erhalten werden. Das entsprechende Equipment zur Messung sollte den Anwender, insbesondere bei seinen Bewegungsmöglichkeiten, nicht wesentlich einschränken, um einen normalen Tagesablauf zu ermöglichen.

Eine Aufgabe ist daher, eine Vorrichtung zur Verfügung zu stellen, die ein bequemes Tragen des Blutdruckmessgerätes ermöglicht und dadurch eine 24h-Messung des Blutdrucks bei normalem Tagesablauf erlaubt.

Als erste Ausführungsform der Erfindung wird ein
System zur Messung des Blutdrucks eines Anwenders zur Verfügung gestellt, umfassend
- eine an einem Oberarm eines Anwenders befestigbare Manschettenvorrichtung mit einem Sensor zur Messung eines Blutdrucks und einer Datenübertragungsvorrichtung zur Übertragung der gemessenen, mit dem Blutdruck des Anwenders assoziierten Daten;
- eine an einem Handgelenk des Anwenders befestigbare Anzeigevorrichtung mit einer Datenempfangsvorrichtung zum Empfang der von der Datenübertragungsvorrichtung übertragenen Daten.
Die mit dem Blutdruck des Anwenders assoziierten Daten können etwa Rohwerte beinhalten, die von einem in der Anzeigevorrichtung vorhandenen Prozessor etwa als Blutdruckwerte angezeigt werden können. Die mit dem Blutdruck des Anwenders assoziierten Daten können ebenfalls die von der Manschettenvorrichtung zur Verfügung aud Rohwerten ermittelte Blutdruckwerte beinhalten. Hierzu muss die Manschettenvorrichtung einen Prozessor aufweisen.

Durch die Übermittlung der Messergebnisse an die Anzeigevorrichtung kann u.a. auf eine Anzeigeeinheit an der Manschettenvorrichtung verzichtet werden. Hierdurch kann ein kompaktes Blutdruckmessgerät zur Verfügung gestellt werden, das sich durch hohen Tragekomfort, da kompakt und gewichtsmäßig leicht, auszeichnet.

Beispielhafte Ausführungsformen werden in den abhängigen Ansprüchen beschrieben.

Gemäß einer beispielhaften Ausführungsform der Erfindung weist die Anzeigevorrichtung ein Armband mit einem Anzeigefeld auf.

Gemäß einer beispielhaften Ausführungsform der Erfindung ist das Anzeigefeld in einem Gehäuse, insbesondere dem Gehäuse einer Uhr, eines Bewegungsmessers oder Pulsuhr, angeordnet.

Ein Bewegungsmesser oder auch Aktivitätssensor ist ein kleines elektronisches Gerät zur Messung und Aufzeichnung körperlicher Bewegung über einen längeren Zeitraum. Die Messung kann auf einem integrierten Beschleunigungssensor basieren, der die Beschleunigung am Körper dort misst, wo der Sensor befestigt ist, nämlich im Falle der Erfindung, am Handgelenk des Anwenders.

Gemäß einer beispielhaften Ausführungsform der Erfindung wird ein System zur Verfügung gestellt, wobei das Mittel zum Übersenden das Messergebnis drahtlos übermittelt oder wobei das Mittel zum Übersenden mit der Uhr mit Leitungen verbunden ist.

Durch eine drahtlose Verbindung kann ein hoher Tragekomfort sichergestellt werden. Bei einer Verbindung mit Leitungen ist eine interferenzfreie Übertragung der Messergebnisse gewährleistet.

In einer weiteren erfindungsgemäßen Ausführungsform wird ein System zur Verfügung gestellt, wobei die Vorrichtung zur Messung als Oberarm-Manschette bzw. als Manschette für ein Handgelenk ausgebildet ist.

Gemäß einem weiteren Ausführungsbeispiel der vorliegenden Erfindung wird ein System zur Verfügung gestellt, wobei die Uhr eine Armbanduhr ist.

Gemäß einem weiteren Ausführungsbeispiel der vorliegenden Erfindung wird ein System zur Verfügung gestellt, wobei die Anzeigevorrichtung eine Bewegungsdetektionseinrichtung zur Aufzeichnung der Aktivität des Anwenders aufweist. Die Bewegungsdetektionseinrichtung kann etwa einen Beschleunigungssensor und/oder einen einfachen Neigungsschalter beinhalten. Mittels der Bewegungsdetektionseinrichtung können die Bewegungen des Anwenders aufgezeichnet werden und beispielsweise für ein internetbasiertes Gesundheitsprogramm verwendet werden, wenn das System über eine Schnittstelle mit dem Internet kommuniziert. Die mittels Sensoren aufgezeichneten Daten können zeigen, wie gesundheitswirksam die gesammelten Aktivitäten waren, insbesondere aber für die Beurteilung der Qualität einer Blutdruckmessung herangezogen werden.

Gemäß einem weiteren Ausführungsbeispiel der vorliegenden Erfindung wird ein System zur Verfügung gestellt, wobei die Anzeigevorrichtung zumindest einen weiteren Sensor zur Aufnahme von Körpersignalen des Anwenders und/oder zur Position des Anwenders und/oder zur aufweist. Der weitere Sensor zur Aufnahme von Körpersignalen des Anwenders kann etwa ein Sensor zur Erfassung des Hautwiderstands des Anwenders, ein Sensor zur Erfassung der Feuchtigkeit der Haut des Anwenders sein. Die Position des Anwenders kann durch bekannte GPS-Sensoren oder durch Luftdruck- und Höhensensoren ermittelt werden. Es versteht sich, dass die Position des Anwenders als Position des Systems angenommen wird. Ferner versteht sich, dass die Position nicht sämtliche Koordinaten beinhalten muss. So wird über den Luftdruck allein die Höhenposition bestimmt.
Durch die Verwendung einer Armbanduhr zur Anzeige der Messergebnisse kann hoher Tragekomfort der Vorrichtung zur Blutdruckmessung und eine jederzeitige Kontrolle der Messergebnisse ermöglicht werden.

Als eine Idee der Erfindung kann angesehen werden, eine Vorrichtung zur Verfügung zu stellen, die eine Blutdruckmessung ermöglicht, die in der Lage ist, ihre Messergebnisse an eine Uhr zur Anzeige der Messergebnisse zu übermitteln. Hierdurch kann die Vorrichtung kompakt aus nur einer Manschette ausgebildet werden und auf eine eigene Anzeigeeinheit verzichtet werden. Die Manschette kann am Oberarm oder am Handgelenk getragen werden.

Die einzelnen Merkmale können selbstverständlich auch untereinander kombiniert werden, wodurch sich zum Teil auch vorteilhafte Wirkungen einstellen können, die über die Summe der Einzelwirkungen hinausgehen.

### KURZE BESCHREIBUNG DER ZEICHNUNGEN

Weitere Einzelheiten und Vorteile der Erfindung werden anhand der in den Zeichnungen dargestellten Ausführungsbeispiele deutlich. Es zeigen
Fig. 1 eine Manschette 1 zur Blutdruckmessung und eine Uhr 2 zur Anzeige der Messergebnisse und
Fig. 2 eine schematische Darstellung möglicher Übermittlungswege für die Messergebnisse der Manschette 1.

### DETAILLIERTE BESCHREIBUNG BEISPIELHAFTER AUSFÜHRUNGSFORMEN

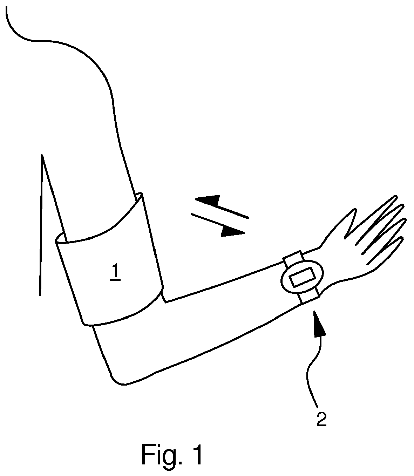

Fig. 1 zeigt eine Manschette 1 zur Blutdruckmessung am Oberarm, wobei die Manschette 1 Messergebnisse an eine Armbanduhr 2 übermitteln kann. Die Armbanduhr 2 kann die Messergebnisse anzeigen. Hierdurch kann auf eine zusätzliche Anzeigeeinheit der Vorrichtung 1 zur Blutdruckmessung verzichtet werden. Die Manschette 1 kann unter einem Hemd bzw. T-Shirt getragen werden. Die Bewegungsmöglichkeit des Anwenders wird wegen der kompakten Bauweise aufgrund des Verzichts auf eine eigene Anzeigeeinheit nicht eingeschränkt. Eine 24h-Messreihe kann somit durchgeführt werden, wobei der Anwender in seinem normalen Tagesablauf nicht oder kaum behindert wird.

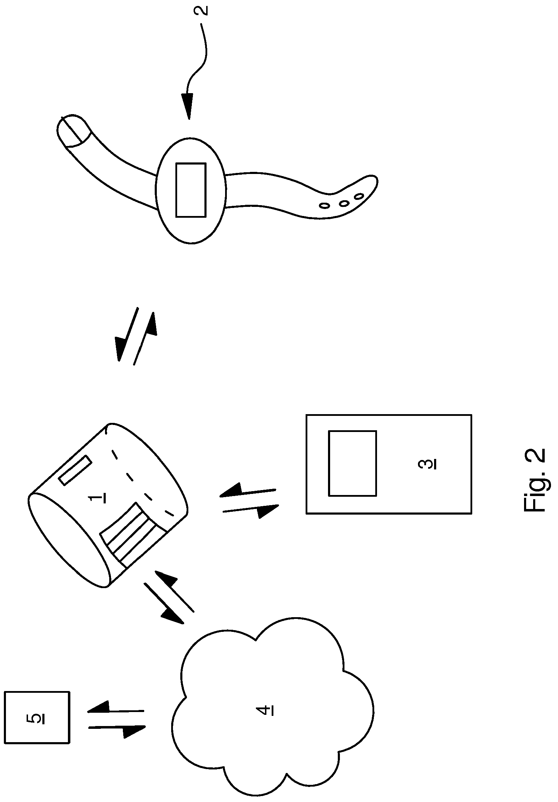

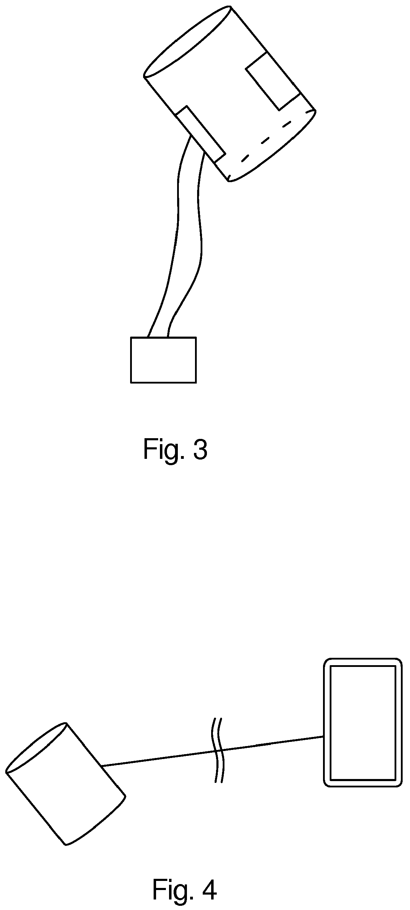

Fig. 2 zeigt die Übermittlungsmöglichkeiten der Vorrichtung 1 zur Blutdruckmessung, die als Manschette 1 ausgebildet ist. Die Manschette 1 kann ihre Messergebnisse an eine Uhr 2, an ein Handy/Smartphone 3 oder ins Internet 4 übermitteln. Via Internet 4 könnte eine Zugriffsmöglichkeit durch einen PC/Computer 5 beispielsweise für ein Krankenhaus und/oder eine Arztpraxis ermöglicht werden.

Es sei angemerkt, dass der Begriff "umfassen" weitere Elemente oder Verfahrensschritte nicht ausschließt, ebenso wie der Begriff "ein" und "eine" mehrere Elemente und Schritte nicht ausschließt.

Die verwendeten Bezugszeichen dienen lediglich zur Erhöhung der Verständlichkeit und sollen keinesfalls als einschränkend betrachtet werden, wobei der Schutzbereich der Erfindung durch die Ansprüche wiedergegeben wird.

### LISTE DER BEZUGSZEICHEN

- 1: Manschette
- 2: Uhr
- 3: Smartphone/Handy
- 4: Internet
- 5: PC/Tablet/Smartphone/Handy

## Patentansprüche

1. System zur Messung des Blutdrucks eines Anwenders umfassend
- eine an einem Oberarm eines Anwenders befestigbare Manschettenvorrichtung (1) mit einem Sensor zur Messung eines Blutdrucks und einer Datenübertragungsvorrichtung zur Übertragung der gemessenen, mit dem Blutdruck des Anwenders assoziierten Daten;
- eine an einem Handgelenk des Anwenders befestigbare Anzeigevorrichtung mit einer Datenempfangsvorrichtung zum Empfang der von der Datenübertragungsvorrichtung übertragenen Daten, wobei die Daten von der Datenübertragungsvorrichtung an die Datenempfangsvorrichtung drahtlos übermittelt werden.

2. System nach Anspruch 1, wobei die Anzeigevorrichtung ein Armband mit einem Anzeigefeld aufweist.

3. System nach Anspruch 2, wobei das Anzeigefeld in einem Gehäuse, insbesondere dem Gehäuse einer Uhr oder Pulsuhr, angeordnet ist.

4. System nach einem der vorgenannten Ansprüche, wobei die Anzeigevorrichtung eine Bewegungsdetektionseinrichtung zur Aufzeichnung der Aktivität des Anwenders aufweist.

5. System nach einem der vorgenannten Ansprüche, wobei die Anzeigevorrichtung zumindest einen weiteren Sensor zur Aufnahme von Körpersignalen des Anwenders und/oder zur Position des Anwenders und/oder zur aufweist.
